# EUROPEAN PATENT APPLICATION

(11) **EP 3 072 514 A1**
(43) Date of publication of application: **28.09.2016**
(21) Application number: 15160648.0
(22) Date of filing: 24.03.2015
(51) Int. Cl.: A61K 31/4439, A61K 31/506, A61P 7/02, A61P 9/00

(54) **Novel oral anticoagulants (NOACs) for preventing development and aggravation of atrial fibrillation**

(71) Applicant: Université Pierre et Marie Curie - Paris 6 (UPMC), 75005 Paris (FR); INSERM (Institut National de la Santé et de la Recherche Médicale), 75654 Paris Cedex 13 (FR); APHP (Assistance Publique - Hôpitaux de Paris), 75001 Paris (FR)
(72) Inventor: Hatem, Stéphane, 75017 Paris (FR); Dufilho, Monique, 75015 Paris (FR); Jumeau, Céline, 75014 Paris (FR)
(74) Representative: Icosa

(57) **Abstract**

The present invention relates to a novel oral anticoagulant (NOAC) for use for preventing development and/or aggravation of atrial fibrillation or complications thereof in a subject with no or low risk of thromboembolism.

## Description

### FIELD OF INVENTION

The present invention relates to the prevention of development and/or of aggravation of atrial fibrillation, and thereby also relates to the prevention of complications of atrial fibrillation. In particular, the present invention relates to the use of a NOAC for preventing the atrial remodeling that promotes the occurrence or maintenance of atrial fibrillation, especially in patients with low risk of thromboembolic complications according to standard scoring systems.

### BACKGROUND OF INVENTION

Atrial fibrillation (AF) is a major threat to healthy ageing in Europe. AF affects 1.5-2% of the European population, and 12-15% of Europe's octogenarians. AF prevalence will double or triple in the next decades. Patients suffering from AF experience reduced life expectancy (with many premature deaths due to heart failure or sudden death) and quality of life, especially at older age. In addition, AF leads to stroke (often with major disability or fatal outcome), cognitive dysfunction, and dementia, and worsens heart failure. Thus, AF has immense detrimental effects on the well-being of elderly European citizens and burdens European health care systems and societies with considerable cost.

AF is most commonly idiopathic in the young, but with increasing age is often associated with heart failure, coronary artery disease, valvular heart disease, cardiomyopathy, diabetes and systemic or pulmonary hypertension. Less common conditions predisposing to AF include myocarditis, pericarditis, pulmonary embolism, pneumonia, lung cancer, hyperthyroidism and drug and alcohol abuse.

Initiation and perpetuation of AF rely on two electrical events in the atrial myocardium: focal ectopy and electrical re-entry. On a molecular and cellular level, several drivers of AF have been identified, including atrial fibrosis, abnormal calcium homeostasis and intracellular calcium leak, ion channel dysfunction (genetic or acquired), and autonomic dysfunction, e.g. in athletes with a high vagal tone.

Atrial fibrillation leads to profound structural alterations, including hypertrophy and dystrophy of cardiomyocytes together with insterstitial fibrosis. Both the presence of AF itself and various clinical conditions that are associated with AF such as hypertension, cardiac hypertrophy, mitral valve diseases or aging favor the structural remodeling of atria, different fibrotic patterns and the formation of AF substrate. This myocardial remodeling is responsible for the impairment of local electrical conduction. Due to this dissociation of electrical activity more three-dimensional conduction events (breakthroughs) occur. Enhanced conduction block and breakthrough both contribute to the increasing complexity of AF in structurally remodeled atria.

Patients with AF are at risk of developing thromboembolic events due to the rapid irregular beating of the heart and blood stasis in poorly contractile left atrium. In particular, AF increases the chance of stroke five-fold. As the consequences of stroke can be devastating, a primary aim of therapy is currently to decrease the risk of arterial thrombus formation and thromboembolism.

Long term anticoagulation therapy is thus recommended for individuals with AF who are considered at moderate to high risk of thromboembolism. These risks include, but are not limited to, age over 65 years, history of previous stroke or transient ischemic attack, hypertension, diabetes, heart failure and the like.

However, as anticoagulation therapy increases the risk of hemorrhage, its benefit-risk ratio is usually considered as too low for patients with no or low risk of thromboembolism. Patients with AF presenting no or low risk of thromboembolism are thus excluded from anticoagulation therapy.

Until 2009, the only available oral anticoagulants for thromboprophylaxis in patients with AF were vitamin K antagonists (VKAs). Novel oral anticoagulants (NOACs) have become alternative options to VKAs for these patients. Indeed, NOACs are associated with the greatest benefits by preventing ischemic stroke, together with a significant reduction in major bleeding, particularly intracranial hemorrhage.

The Applicant surprisingly showed that NOAC administration may prevent or even reduce atrial remodeling associated with AF, acting in particular as an antihypertrophic and anti-fibrotic agent. These results highlight the possibility to extend the therapeutic use of anticoagulant agents to all AF patients, and in particular to substantially healthy AF patients, for example to patients with no or low risk of stroke.

### SUMMARY

One object of the invention is a NOAC for use in preventing development and/or aggravation of atrial fibrillation (AF) in a subject.

In one embodiment AF is de novo AF.

In another embodiment, the subject is substantially healthy.

In another embodiment, the subject has no or low risk of thromboembolism, preferably the subject as a CHADS₂ score or a CHA₂DS₂-VASC score of 0.

In another embodiment, said NOAC is a direct thrombin inhibitor.

In another embodiment, said NOAC is selected from dabigatran or a derivative or a salt or solvate thereof and S35972.

In another embodiment, said NOAC is dabigatran etexilate.

In another embodiment, said NOAC is orally administered.

Another object of the invention is a pharmaceutical composition for use in preventing development and/or aggravation of AF in a subject, wherein said pharmaceutical composition comprises at least one NOAC as described here above in combination with at least one pharmaceutically acceptable excipient.

Another object of the invention is a medicament for use in preventing development and/or aggravation of AF in a subject, wherein said pharmaceutical composition comprises at least one NOAC as described here above.

Another object of the invention is a method for preventing development and/or aggravation of AF in a subject, wherein said method comprises administering a therapeutically effective amount of at least one NOAC to the subject.

Another object of the invention is a method for preventing myocardial remodeling, preferably atrial remodeling, thereby preventing development and/or aggravation of AF, wherein said method comprises administering a therapeutically effective amount of at least one NOAC to the subject.

In one embodiment, said myocardial remodeling is an increase of atrial area, an increase of atrial diameter, an increased atrial expression of hypertrophic and fibrotic markers.

### DEFINITIONS

In the present invention, the following terms have the following meanings:
- "**NOAC**" (standing for Novel Oral Anticoagulant) refers to the therapeutic class of new target-specific oral anticoagulants, which are not vitamin K antagonists (VKAs). The NOACs, which are now readily available for use, includes two pharmacological groups: direct thrombin inhibitors (DTI) and inhibitors of activated factor X (factor Xa).
- "**Prevention**": refers to preventing or avoiding the occurrence of symptom. In the present invention, the term "prevention" may refer to a secondary prevention, i.e. to the prevention of the re-occurrence of a symptom or a relapse of the disease.
- "**Therapeutically effective amount**" means level or amount of NOAC that is aimed at delaying or preventing the onset of AF or a complication thereof, without causing significant negative or adverse side effects to the target.
- "**Subject**" refers to a warm-blooded animal, more preferably a human. Preferably, the subject is a patient, i.e. the subject is awaiting the receipt of, or is receiving medical care or is/will be the object of a medical procedure.
- "**Pharmaceutically acceptable excipient**" refers to an excipient that does not produce an adverse, allergic or other untoward reaction when administered to an animal, preferably a human. It includes any and all solvents, dispersion media, coatings, antibacterial and antifungal agents, isotonic and absorption delaying agents and the like. For human administration, preparations should meet sterility, pyrogenicity, general safety and purity standards as required by regulatory offices, such as, for example, FDA Office or EMA.
- "**About**" preceding a figure means plus or less 10% of the value of said figure.

### DETAILED DESCRIPTION

The present invention relates to a method for preventing development and/or aggravation of AF and complications thereof in a subject, comprising administering to the subject a therapeutically effective amount of at least one NOAC.

Indeed, the Applicant demonstrated that the administration of at least one NOAC prevents the development of the arrhythmogenic substrate of atrial fibrillation. In particular, the administration of NOAC prevents the atrial remodeling that promotes the occurrence or maintenance of atrial fibrillation. In one embodiment, the method of the invention is for preventing or reducing atrial hypertrophy and fibrosis associated with AF, thereby preventing development and/or aggravation of AF.

In one embodiment, AF is de novo AF (also known as first detected AF), i.e. the subject developed only one episode of AF.

In another embodiment, AF is paroxysmal AF. Paroxysmal AF may be defined as recurrent episodes of AF that stop on their own in less than 7 days, preferably in less than 48 hours.

In another embodiment, AF is persistent AF. Persistent AF may be defined as recurrent episodes that last more than 7 days, or requiring a cardioversion for stopping. As used herein, the term "cardioversion" refers to a noninvasive conversion of an irregular heartbeat to a normal heartbeat using electrical (e.g. DC electrical shock) or chemical means (e.g. using drugs selected in the group of amiodarone, dronedarone, procainamide, dofetilide, ibutilide, propafenone, flecainide, vernakalant).

In another embodiment, AF is permanent AF. Permanent AF may be defined as an ongoing long-term episode of AF, with no impact of cardioversion.

In one embodiment, AF is idiopathic AF.

In one embodiment, the complication of AF is thromboembolism, heart failure or aggravation of pre-existing heart failure.

In one embodiment, said NOAC is a direct thrombin inhibitor (thrombin is also known as factor IIa).

Examples of direct thrombin inhibitors (DTI) that may be used in the present invention include, but are not limited to, dabigatran or a derivative or a salt or solvate thereof (preferably dabigatran etexilate), S35972, AZD0837 and Ximelagatran.

In one embodiment, the anticoagulant is dabigatran or a salt or solvate thereof (preferably dabigatran etexilate).

In another embodiment, the anticoagulant is S35972 having the following structure:

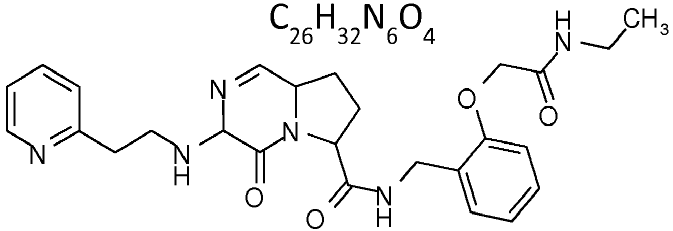

S35972 was described in Rupin et al (Journal of Thrombosis and Haemostasis, 2011, 9: 1375-1382).

In one embodiment, the NOAC is an inhibitor of factor Xa. Examples of inhibitors of factor Xa include, but are not limited to, Rivaroxaban, Apixaban, Edoxaban, Betrixaban, Darexaban.

The present invention also relates to a composition comprising at least one NOAC, preferably a direct thrombin inhibitor, more preferably dabigatran or a derivative or a salt or solvate thereof (preferably dabigatran etexilate) or S35972. In one embodiment, the composition comprises a therapeutically effective amount of at least one NOAC.

The present invention also relates to a pharmaceutical composition comprising at least one NOAC, preferably a direct thrombin inhibitor, more preferably dabigatran or a derivative or a salt or solvate thereof (preferably dabigatran etexilate) or S35972, in combination with at least one pharmaceutically acceptable excipient. In one embodiment, the pharmaceutical composition comprises a therapeutically effective amount of at least one NOAC.

The present invention also relates to a medicament comprising at least one NOAC, preferably a direct thrombin inhibitor, more preferably dabigatran or a derivative or a salt or solvate thereof (preferably dabigatran etexilate) or S35972. In one embodiment, the medicament comprises a therapeutically effective amount of at least one NOAC.

The present invention also relates to a composition, pharmaceutical composition or medicament of the invention for preventing development and/or aggravation of AF or complications thereof in a subject.

In one embodiment, the subject is affected, preferably is diagnosed with de novo AF (also known as first detected AF), i.e. the subject developed only one episode of AF.

In another embodiment, the subject developed at least one episode of AF, preferably one, two or three episodes of AF.

In another embodiment, the subject is affected, preferably is diagnosed with paroxysmal AF.

In another embodiment, the subject is affected, preferably is diagnosed with persistent AF.

In another embodiment, the subject is affected, preferably is diagnosed, with permanent AF.

In one embodiment, the subject is affected, preferably is diagnosed, with idiopathic AF.

In one embodiment, the subject is substantially healthy. In the present invention, the term "substantially healthy subject" relates to a subject who has not been previously diagnosed or identified as having or suffering from or who is not at risk of structural heart diseases (such as, for example, hypertension, diabetes mellitus, valve diseases, ischemic cardiopathies).

In one embodiment, the subject presents no or low risk of AF complications. In one embodiment, the subject presents no or low risk of thromboembolism, heart failure or aggravation of pre-existing heart failure.

Scoring systems for assessing the risk of thromboembolism of patients with AF are known in the art, and include, but are not limited to, CHADS₂ and CHA₂DS₂-VASC.

CHADS₂ and CHA₂DS₂-VASC are two scoring system well known in the art based on the mathematical combination within an addition of weighted risk factors, as shown in the Table 1 below:

**Table 1: Comparison of CHADS₂ and CHA₂DS₂-VASC**

| Risk factor | CHADS₂ | CHA₂DS₂-VASC |
|---|---|---|
| Congestive heart failure/left ventricular dysfunction | 1 | 1 |
| **H**ypertension | 1 | 1 |
| **A**ge ≥ 75 yrs | 1 | 2 |
| **D**iabetes mellitus | 1 | 1 |
| **S**troke / transient ischemic attack / thromboembolism | 2 | 2 |
| **V**ascular disease (previous myocardial infarction, peripheral artery disease, aortic plaque) | NA | 1 |
| **A**ge 65-74 yrs | NA | 1 |
| Sex category (female) | NA | 1 |

| | | |
|---|---|---|
| NA: Not applicable | | |

In one embodiment, the subject of the invention has a CHADS₂ score of 0 or 1, preferably a CHADS₂ score of 0.

In one embodiment, the subject of the invention has a CHA₂DS₂-VASC score of 0 or 1, preferably a CHA₂DS₂-VASC score of 0.

In one embodiment, the subject has a high bleeding risk. In another embodiment, the subject has a moderate bleeding risk. In another embodiment, the subject has a low bleeding risk.

Bleeding risk may be assessed in subjects with AF using a scoring system known in the art, including, without limitation, the HAS-BLED score, wherein HAS-BLED stands for Hypertension, Abnormal Renal/Liver Function, Stroke, Bleeding History or Predisposition, Labile INR, Elderly, Drugs/Alcohol Concomitantly.

HAS-BLED is a scoring system well known in the art based on the mathematical combination within an addition of weighted risk factors, as shown in the Table 2 below:

**Table 2: HAS-BLED score**

| Risk factor | HAS-BLED |
|---|---|
| Hypertension | 1 |
| Abnormal Renal/Liver Function | 1 for each |
| Stroke | 1 |
| Bleeding History or Predisposition | 1 |
| Labile INR | 1 |
| Elderly | 1 |
| Drugs/Alcohol Concomitantly | 1 for each |

According to the HAS-BLED score, a low bleeding risk corresponds to a score of 0, a moderate risk corresponds to a score of 1 or 2, and a high bleeding risk corresponds to a score of 3 or more.

In one embodiment, the subject has a HAS-BLED score ranging from 0 to 6, preferably from 0 to 3. Preferably, the subject has a HAS-BLED score ranging from 0 to 6, preferably from 0 to 3, wherein preferably the Abnormal Renal/Liver Function score, the Stroke score and the Elderly score are of 0.

Therefore, in one embodiment, the subject has a CHADS₂ score of 0 or 1, preferably a CHADS₂ score of 0, and a HAS-BLED score ranging from 0 to 6, preferably ranging from 0 to 3, wherein preferably the Abnormal Renal/Liver Function score, the Stroke score and the Elderly score are of 0.

In another embodiment, the subject has a CHA₂DS₂-VASC score of 0 or 1, preferably a CHA₂DS₂-VASC score of 0, and a HAS-BLED score ranging from 0 to 6, preferably ranging from 0 to 3, wherein preferably the Abnormal Renal/Liver Function score, the Stroke score and the Elderly score are of 0.

In one embodiment, the subject has a CHADS₂ score of 0 or 1, preferably a CHADS₂ score of 0, and a HAS-BLED score of 0. In another embodiment, the subject has a CHADS₂ score of 0 or 1, preferably a CHADS₂ score of 0, and a HAS-BLED score of 1 or 2. In another embodiment, the subject has a CHADS₂ score of 0 or 1, preferably a CHADS₂ score of 0, and a HAS-BLED score of 3 or more.

In one embodiment, the subject has a CHA₂DS₂-VASC score of 0 or 1, preferably a CHA₂DS₂-VASC score of 0, and a HAS-BLED score of 0. In another embodiment, the subject has a CHA₂DS₂-VASC score of 0 or 1, preferably a CHA₂DS₂-VASC score of 0, and a HAS-BLED score of 1 or 2. In another embodiment, the subject has a CHA₂DS₂-VASC score of 0 or 1, preferably a CHA₂DS₂-VASC score of 0, and a HAS-BLED score of 3 or more.

In one embodiment, the subject is a male. In one embodiment, the subject is a female.

In one embodiment, the at least one NOAC is orally administered. Examples of forms adapted for oral administration include, but are not limited to, tablets, orodispersing/orally disintegrating tablets, effervescent tablets, powders, granules, pills (including sugarcoated pills), dragees, capsules (including soft gelatin capsules), syrups, liquids, gels or other drinkable solutions, suspensions, slurries, liposomal forms and the like.

In one embodiment, the at least one NOAC is dabigatran etexilate and is orally administered.

In one embodiment, the at least one NOAC is S35972 and is orally administered.

In one embodiment, the at least one NOAC is administered twice a day, once a day, once every two days, twice a week, once a week, or once every 1, 2, 3, 4, 5, 6, 7 weeks or more. In one embodiment, the NOAC is administered for at least one month, preferably at least two months or more.

In one embodiment, the therapeutically effective amount of the at least one NOAC ranges from about 10 to about 1000 mg, preferably from about 20 to about 600 mg, more preferably from about 30 mg to about 600 mg. In one embodiment, the therapeutically effective amount of the at least one NOAC is of about 300 mg. In another embodiment, the therapeutically effective amount of the at least one NOAC is of about 440 mg.

In one embodiment, the therapeutically effective amount of the at least one NOAC ranges from about 10 to about 500 mg twice a day, preferably from about 20 to about 400 mg twice a day, more preferably from about 30 mg to about 300 mg twice a day. In one embodiment, the therapeutically effective amount of the at least one NOAC is of about 150 mg twice a day. In another embodiment, the therapeutically effective amount of the at least one NOAC is of about 220 mg twice a day.

In one embodiment, the at least one NOAC is dabigatran etexilate and the therapeutically effective amount of dabigatran etexilate ranges from about 100 to about 1000 mg/day, preferably from about 200 to about 750 mg/day, more preferably from about 280 mg/day to about 600 mg/day. In one embodiment, the therapeutically effective amount of dabigatran etexilate is of about 300 mg/day. In another embodiment, the therapeutically effective amount of dabigatran etexilate is of about 440 mg/day.

In one embodiment, the at least one NOAC is dabigatran etexilate, and the therapeutically effective amount of dabigatran etexilate ranges from about 50 to about 300 mg BID, preferably from about 150 mg to about 300 mg BID (wherein BID stands for "bis in die", i.e. twice a day).

In one embodiment, the at least one NOAC is S35972 and the therapeutically effective amount of S35972 ranges from about 20 to about 800 mg/day, preferably from about 40 to about 480 mg/day, more preferably from about 120 mg/day to about 240 mg/day.

In one embodiment, the at least one NOAC is administered at a dose such that the plasmatic concentration of the at least one NOAC reaches about 50 to 200 ng/ml, preferably about 60 to about 150 ng/ml, more preferably about 91 ng/ml.

In one embodiment, dabigatran etexilate is administered at a dose such that the plasmatic concentration of dabigatran etexilate reaches about 50 to 200 ng/ml, preferably about 60 to about 150 ng/ml, more preferably about 91 ng/ml.

In one embodiment, S35972 is administered at a dose such that the plasmatic concentration of S35792 reaches about 50 to 200 ng/ml, preferably about 60 to about 150 ng/ml, more preferably about 90 to 100 ng/ml.

The present invention further relates to a method for preventing myocardial remodeling, preferably atrial remodeling, thereby preventing development and/or aggravation of AF and thereby preventing complications thereof in a subject, wherein said method comprises administering a therapeutically effective amount of at least one NOAC to the subject.

In one embodiment, the method of the invention has no impact on ventricle function.

Myocardial remodeling associated with AF and that may be prevented by the method of the invention include, but is not limited to, increase of atrial surface, increase of atrial diameter, an increased atrial expression of hypertrophic and fibrotic markers.

Indeed, the Applicant demonstrated in the Examples that the administration of at least one NOAC in a patient with AF results in a decrease of atrial surface, a decrease of atrial diameter, and prevention of atrial expression of hypertrophic and fibrotic markers.

Therefore, the present invention further relates to a method for decreasing or for preventing atrial dilatation, thereby preventing development and/or aggravation of AF and thereby preventing complications thereof in a subject, wherein said method comprises administering a therapeutically effective amount of at least one NOAC to the subject.

Examples of hypertrophic markers include, but are not limited to, β isoform of the heavy chain of myosin (β-MHC), brain natriuretic peptide BNP, and atrial natriuretic peptide ANP.

A non-limiting example of fibrotic markers is HB-EGF and plasminogen activator inhibitor 1 (PAI-1), which has also a role in thrombogenesis.

The present invention further relates to a method for reducing, or for preventing the increase of the expression of β-MHC mRNA in the atrium and/or in the ventricle, the expression of BNP in the atrium and/or in the ventricle, the expression of ANP in the ventricle, the expression of PAI-1 in the atrium and/or the expression of HB-EGF in the atrium and/or in the ventricle, thereby preventing development and/or aggravation of AF and thereby preventing complications thereof in a subject, wherein said method comprises administering a therapeutically effective amount of at least one NOAC to the subject.

Atrial remodeling is composed of cellular hypertrophy and fibrosis.

Therefore, the present invention further relates to a method for inhibiting or reducing or preventing myocardial hypertrophy in a subject, wherein said method comprises administering a therapeutically effective amount of at least one NOAC to the subject. The present invention further relates to a method for inhibiting or reducing or preventing myocardial fibrosis in a subject, wherein said method comprises administering a therapeutically effective amount of at least one NOAC to the subject.

The present invention further relates to a method for reducing the duration of an episode of AF associated with structural remodeling of the atria, wherein said method comprises administering a therapeutically effective amount of at least one NOAC to the subject.

### BRIEF DESCRIPTION OF THE DRAWINGS

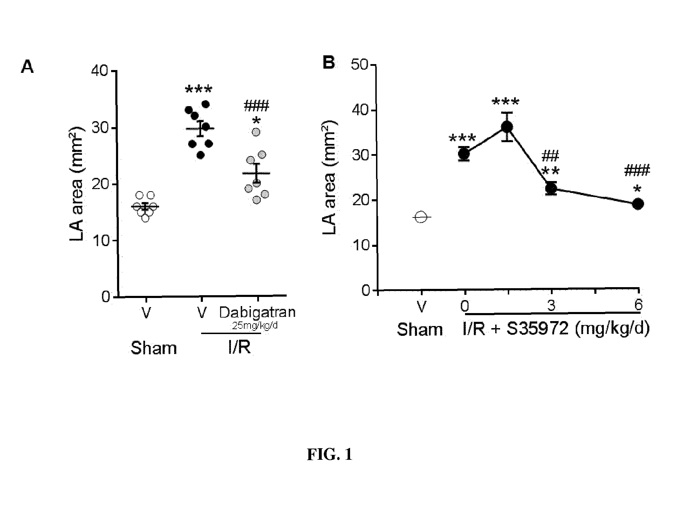

**Figure 1** is a combination of two graphs showing left atrium (LA) area after one month of treatment with DTI Dabigatran (A) or S35792 (B). Vehicle (V) is 60% H₂O/40% PEG 300 for dabigatran and saline for S35972. Treatment of rats started just after thoracotomy without (Sham) or with occlusion of left coronary artery followed by reperfusion (I/R). Results are presented as mean +/- S.E.M, **P*<0.05, ***P*<0.01 and *** *P*<0.001 vs Sham; ^{##}*P*<0.01 and ^{###}*P<*0.001 vs untreated I/R.

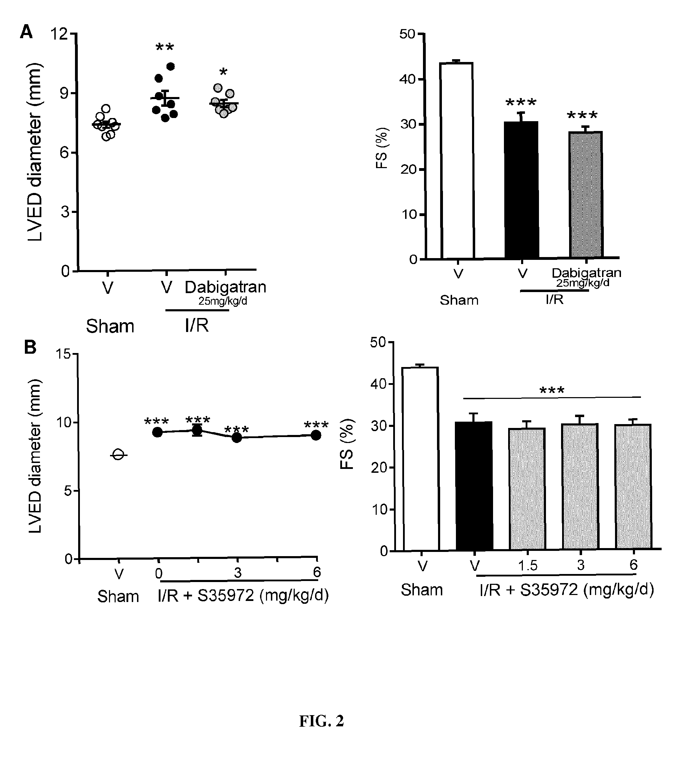

**Figure 2** is a combination of graphs showing left ventricular end-diastolic (LVED) diameter and fractional shortening (FS) after one month of treatment with Dabigatran (A) or S35792 (B). Vehicle (V) is 60% H₂O/40% PEG 300 for dabigatran and saline for S35972. Treatment of rats started just after thoracotomy without (Sham) or with occlusion of left coronary artery followed by reperfusion (I/R). Results are presented as mean +/- S.E.M. * P<0.05; ** P<0.001, *** P<0.001 vs Sham; #P<0.05 vs untreated I/R.

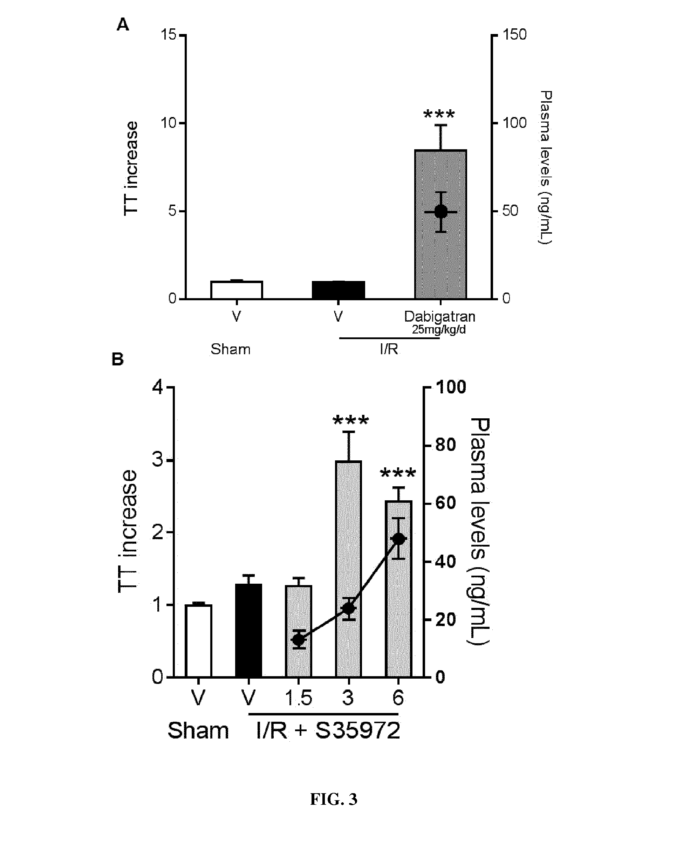

**Figure 3** is a combination of two graphs showing thrombin time (TT), which reflects anticoagulant activity, and plasma concentrations of Dabigatran (A) and S35792 (B) in rats after throracotomy with occlusion of left coronary artery followed by reperfusion (I/R). Vehicle (V) is 60% H₂O/40% PEG 300 for dabigatran and saline for S35972. Tests were carried out on blood collected by cardiac puncture at sacrifice (i.e. for Dabigatran, 90 minutes after last oral administration). DTI plasma levels are quantified using LC-MS/MS. TT are expressed as mean +/- S.E.M with reference to the mean value of sham animals that underwent thoracotomy only. *** P<0.001 vs untreated I/R.

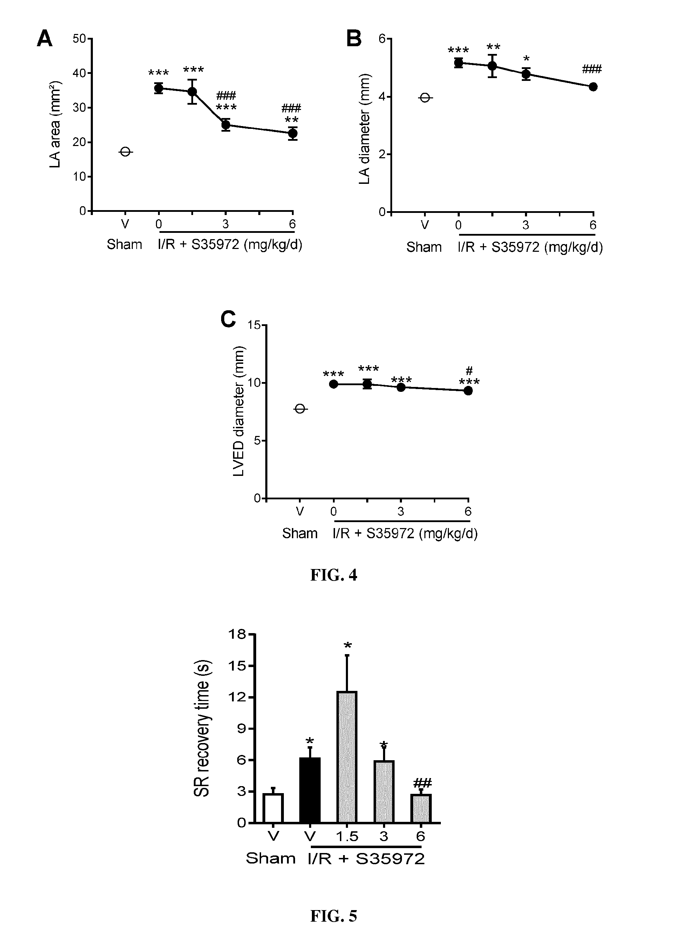

**Figure 4** is a combination of three graphs showing left atrium (LA) area (A) and diameter (B) and left ventricle end-diastolic (LVED) diameter (C) after two months of treatment with S35972. Treatment of rats started just after thoracotomy without (Sham) or with occlusion of left coronary artery followed by reperfusion (I/R). LA area was measured by bidimensional echocardiography. LA and LVED diameters were measured in time-motion mode. Results are presented as mean +/- S.E.M. *P<0.05, ***P*<0.01 and *** *P*<0.001 vs Sham; ^{#}*P*<0.05 ^{###}*P*<0.001 vs untreated I/R.
**Figure 5** is a graph showing the impact of S35972 administration during two months on the duration of an episode of AF in rats undergone thoracotomy without (Sham) or with occlusion of left coronary artery followed by reperfusion (I/R). AF duration is measured as the time between the end of the electric stimulation and the return to sinusoidal rhythm. Results are presented as mean +/- S.E.M, **P*<0.05 vs Sham; ^{##}*P*<0.01 vs untreated I/R,

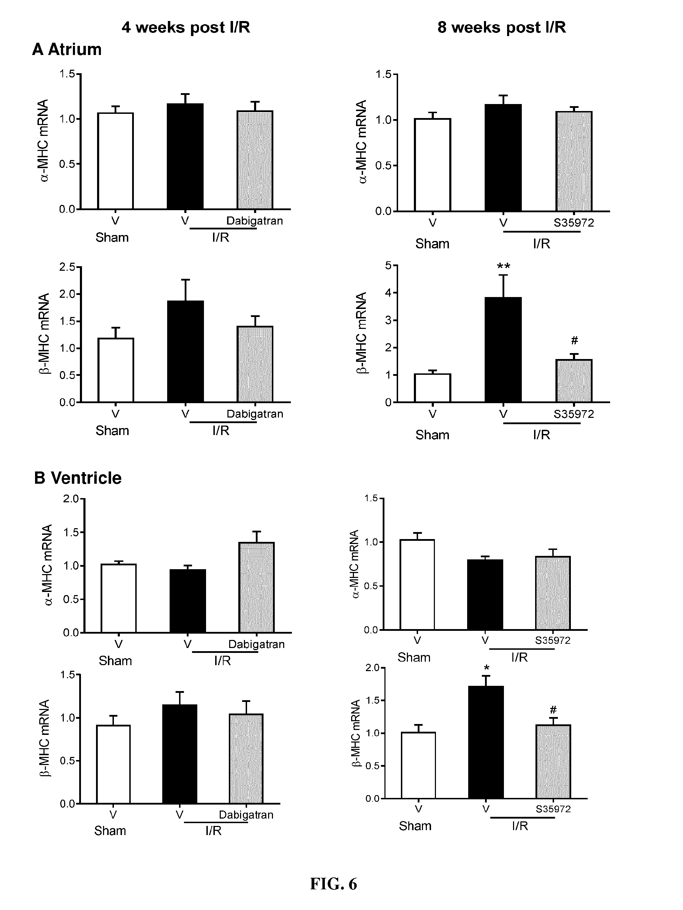

**Figure 6** is a combination of graphs showing the impact of treatments with 25 mg/kg/d dabigatran for 4 weeks and 6 mg/kg/d S35972 for 8 weeks on atrial (A) and ventricular (B) expression of myosin heavy chain (MHC) isoforms. Rats undergone thoracotomy without (Sham) or with occlusion of left coronary artery followed by reperfusion (I/R). Expression of mRNA was measured with real-time qPCR. Results are presented as mean +/- S.E.M. *P<0.05, **P<0.01 vs Sham; #P<0.05 vs untreated I/R.

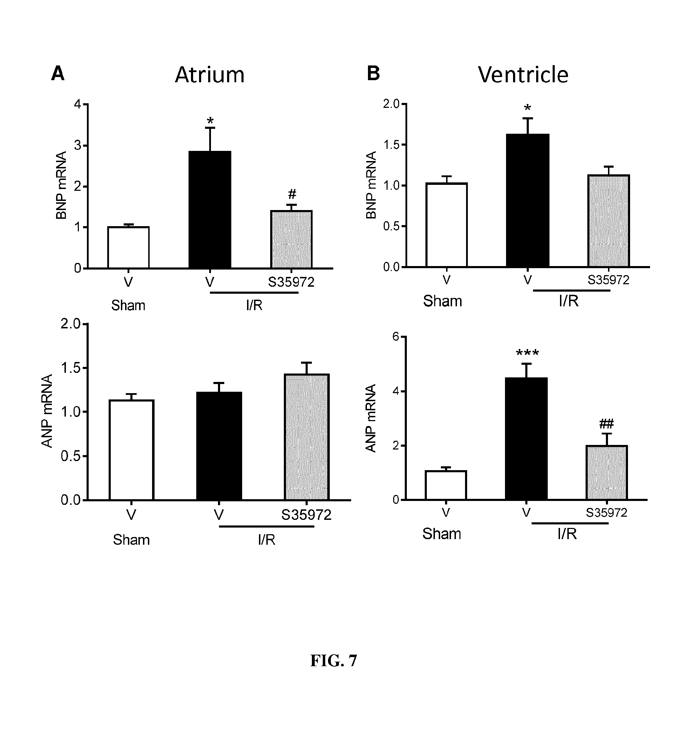

**Figure 7** is a combination of graphs showing the impact of 2 months S35972 administration on the expression of the hypertrophic markers, brain and atrial natriuretic peptides (BNP and ANP) in atria (A) and ventricle (B). Rats undergone thoracotomy without (Sham) or with occlusion of left coronary artery followed by reperfusion (I/R). Expression of mRNA was measured with real-time qPCR. Results are presented as mean +/- S.E.M. *P<0.05, ***P<0.001 vs Sham; #P<0.05, ##P<0.01 vs untreated I/R.

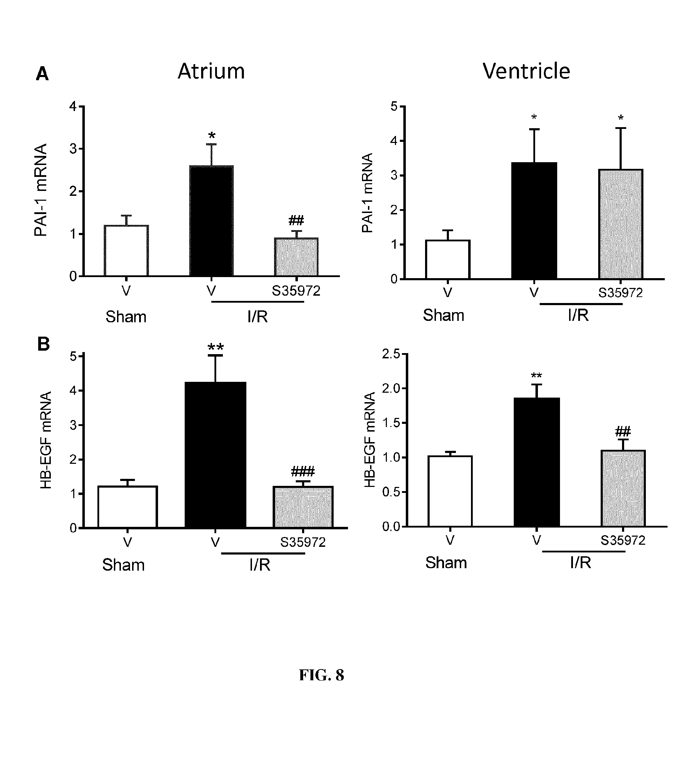

**Figure 8** is a combination of graphs showing the impact of 2 months S35972 administration on the expression of fibrotic markers, plasminogen activator inhibitor 1 (PAI-1, A) and heparin-binding epidermal growth factor (HB-EGF, B) in atria and ventricle. Rats undergone thoracotomy without (Sham) or with occlusion of left coronary artery followed by reperfusion (I/R). Expression of mRNA was measured with real-time qPCR. Results are presented as mean +/- S.E.M. *P<0.05, ***P<0.001 vs Sham; #P<0.05, ##P<0.01 vs untreated I/R.

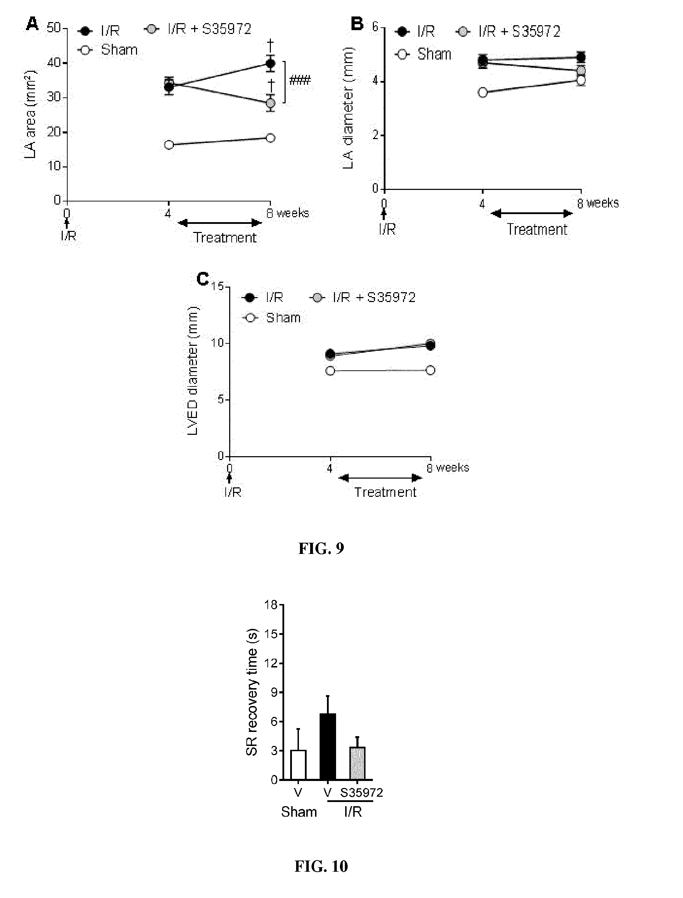

**Figure 9** is a combination of graphs showing left atrium (LA) area (A) and diameter (B) and left ventricle end-diastolic (LVED) diameter (C) after one month of treatment with S35972. Treatment of rats started one month post thoracotomy without (Sham) or with occlusion of left coronary artery followed by reperfusion (I/R). LA area was measured by bidimensional echocardiography. LVED and LA diameters were measured in time-motion mode. Results are presented as mean +/- S.E.M, *^{###}P<*0.001 vs untreated I/R; †*P*<0.05 vs I/R rats before treatment with S35972 or vehicle.
**Figure 10** is a graph showing the impact of of S35972 administered 1 month post I/R on duration of AF. Rats undergone thoracotomy without (Sham) or with occlusion of left coronary artery followed by reperfusion (I/R). AF duration is measured as the time between the end of the electric stimulation and return to sinusoidal rhythm. Results are presented as mean +/- S.E.M.

### EXAMPLES

The present invention is further illustrated by the following examples.

### Pre-clinical pharmacological experiments

***Model*:** Male OFA Sprague-dawley rats weighting 200-220g were obtained from Charles River (L'Arbresle, France) and housed for two weeks in CEF 105B of Pierre and Marie Curie University (Paris, France) before experiments. All experimental procedure were reviewed by the local Ethical Committee for animal research and approved by the French Ministry of Education and Research (authorization N°00429.03).

Animals were anesthetized with 30 mg/kg sodium pentobarbital, and then intubated and ventilated at 60 respirations/min while body temperature was maintained at 37°C. Rats underwent thoracotomy and their left coronary artery was either left intact (Sham) or occluded with a 4.0 silk suture at 1 mm from the artery origin. Ischemia was maintained for 30 min and the clamp was removed to initiate a definitive reperfusion phase. This episode of ischemia/reperfusion (I/R) induced a progressive dilatation of ventricle, then of atria.

***Products:*** The DTIs, Dabigatran etexilate and S35972 (dihydrochloride salt of 4-oxo-3-(2-pyridin-2-ylethylamino)-4,6,7,8-tetrahydro-pyrrolo[1,2-a]pyrazine-6-carboxylic acid 2-ethylcarbamoyl methoxy-benzylamide (Rupin et al, 2011) were synthesized. S35972 was dispensed with an ALZET® osmotic minipump subcutaneously implanted following I/R during anesthesia. Rats received 1.5, 3 6 or 15 mg/kg/d of S35972 or vehicle (Saline) during 4 or 8 weeks. Dabigatran etexilate at 12.5 mg/kg or vehicle (PEG 300 40%/H2O 60%) was administered by force-feeding twice daily during 4 weeks. Treatment started one hour after I/R procedure, when rats regained consciousness. For dabigatran, blood sampling is performed 90 minutes after force-feeding. Plasma concentrations of Dabigatran and S35972 are determined by liquid chromatography-tandem mass spectrometry (LCMS/MS) with the protocol used for the detection of Dabigatran in human plasma.

### Results:

Four weeks post-I/R, echocardiographic evaluation shows that both products (S35972 and dabigatran) prevent the dilatation of left atrium (see **Figure 1**).

As shown in Figure 2, both products (S35972 and dabigatran) do not impact ventricular diameter and function (**Figure 2**).

When administered at a dose of 25 mg/kg/day (i.e. 12.5 mg/kg BID), the plasma concentration of dabigatran reaches 49.2 ± 19.3 (SD) ng/ml and the thrombin time increases by a factor 8, which confirms a strong anticoagulant activity (**Figure 3A**). When administered at a dose of 3 or 6 mg/kg/day, the plasma concentration of S35975 reaches 24.1 ± 5.7 and 57.2±24.4 ng/ml, respectively. The thrombin time increases by a factor 3 for the two doses (**Figure 3B**).

The results presented in **Figures 1** to **3** thus demonstrate that dabigatran and S35972 inhibit both blood coagulation and atrial dilatation.

Administration of S35972 for 8 weeks at a dose of either 3 or 6 mg/kg/day decreases the left atrium area (**Figure 4A**). However, only a dose of 6 mg/kg/day significantly reduces atrial and ventricular diameters (see **Figures 4B and C**).

Eight weeks post I/R, a rapid stimulation of left atrium with an electrode introduced in the esophagus induces an episode of paroxysmal AF. Administration of 6 mg/kg/day of S35972 decreases the duration of this episode of paroxysmal AF to the control values (**Figure 5****).**

These results thus demonstrate that chronic treatment with S35972 decrease atrial dilatation and AF duration.

In tissues, dilatation of the left atrium is associated with reprogramming of fetal genes , such as, for example, β-MHC, as shown in **Figure 6A****.** Administration of 25 mg/kg/day of dabigatran or of 6 mg/kg/day of S35972 prevents the synthesis of β-MHC without impacting the expression of α-MHC, either 4 or 8 weeks post I/R (see **Figure 6A**).

In the ventricle, I/R also induces the synthesis of β-MHC, while α-MHC remains constant, as shown in **Figure 6B****.** Administration of 6 mg/kg/day of S35972 prevents the synthesis of β-MHC without significant impact on the expression of α-MHC, either 4 or 8 weeks post I/R (see **Figure 6B**).

Moreover, I/R-induced reprogramming of fetal genes is associated with an induction of the synthesis of BNP in atria and ANP in ventricles, (see **Figure 7**). Administration of 6 mg/kg/day of S35972 prevents the synthesis of BNP both in the atrium and in the ventricle. Regarding ANP, S35972 administration does not impact its expression in the atrium, but significantly reduces its synthesis in the ventricle (**see** **Figure 7**).

These results thus demonstrate that dabigatran and S35972 inhibit the hypertrophic response of myocardial atrium and ventricle to I/R.

Atrial remodeling is composed of cellular hypertrophy and modifications of extracellular matrix proteins that triggers fibrosis. PAI-1 is an extracellular matrix protein, which has a dual role by contributing to pathogenesis of thrombotic events and to cardiac remodeling. The mRNA expression of PAI-1 is amplified in both dilated atria and ventricles, but treatment with S35972 has an inhibitory effect in atria only (**Figure 8A**), in agreement with the little effect of S35972 on LVED diameter. The expression of HB-EGF, which is a potent inductor of extracellular matrix remodeling, is also amplified in dilated atria and ventricles. Treatment with S35972 prevents the induction of HB-EGF mRNA in both atria and ventricles (**Figure 8B**). This could participate in the observed effect of on LVED diameter.

These results demonstrate that S35972 inhibits the atrial expression of extracellular matrix protein and growth factors induced by I/R.

The power of S35972 to regress already established atrial dilatation was tested by starting treatment one month after the I/R episode (**Figure 9**). Administration of 6 mg/kg/d during one month significantly reduced the left atrium area (**Figure 9A**), but not the diameter, which decreased to an intermediate value between Sham and I/R rats (**Figure 9B**). S35972 had no effect on left ventricle end-diastolic diameter (**Figure 9C**).

Moreover, administration of 6 mg/kg/day S35972 in rats with dilated atria tends to decrease AF duration (**Figure 10**).

These results thus demonstrate that direct inhibitors of thrombin (such as, for example, dabigatran and S35972) inhibit the development of atrial dilatation and remodeling and the susceptibility to AF, and decrease atrial dilatation.

## Claims

1. NOAC for use in preventing development and/or aggravation of atrial fibrillation (AF) in a subject.

2. NOAC for use according to claim **1**, wherein AF is de novo AF.

3. NOAC for use according to claim **1** or **2**, wherein the subject is substantially healthy.

4. NOAC for use according to anyone of claims **1** to **3**, wherein the subject has no or low risk of thromboembolism, preferably the subject as a CHADS₂ score or a CHA₂DS₂-VASC score of 0.

5. NOAC for use according to anyone of claims **1** to **4**, wherein said NOAC is a direct thrombin inhibitor.

6. NOAC for use according to anyone of claims **1** to **5**, wherein said NOAC is selected from dabigatran or a derivative or a salt or solvate thereof and S35972.

7. NOAC for use according to anyone of claims **1** to **6**, wherein said NOAC is dabigatran etexilate.

8. NOAC for use according to anyone of claims **1** to **7**, being orally administered.

9. Pharmaceutical composition for use in preventing development and/or aggravation of AF in a subject, wherein said pharmaceutical composition comprises at least one NOAC for use according to anyone of claims **1** to **8** in combination with at least one pharmaceutically acceptable excipient.

10. Medicament for use in preventing development and/or aggravation of AF in a subject, wherein said pharmaceutical composition comprises at least one NOAC for use according to anyone of claims **1** to **8.**

11. Method for preventing development and/or aggravation of AF in a subject, wherein said method comprises administering a therapeutically effective amount of at least one NOAC to the subject.

12. Method for preventing myocardial remodeling, preferably atrial remodeling, thereby preventing development and/or aggravation of AF, wherein said method comprises administering a therapeutically effective amount of at least one NOAC to the subject.

13. Method according to claim **12,** wherein said myocardial remodeling is an increase of atrial area, an increase of atrial diameter, an increased atrial expression of hypertrophic and fibrotic markers.
